# EUROPEAN PATENT APPLICATION

(11) **EP 4 534 682 A1**
(43) Date of publication of application: **09.04.2025**
(21) Application number: 23812118.0
(22) Date of filing: 23.05.2023
(51) Int. Cl.: C12N 15/87

(54) **MESSENGER RNA CARRIER BASED ON NANOPARTICLE-OLIGO T CONJUGATE**

(30) Priority: 24.05.2022 KR 20220063493
(71) Applicant: NES BIOTECHNOLOGY CO., LTD., Seoul 06974 (KR)
(72) Inventor: LEE, Kangseok, Gwangju-si, Gyeonggi-do 12765 (KR); BAE, Jeehyeon, Gwangju-si, Gyeonggi-do 12765 (KR); YEOM, Jihyun, Yangju-si, Gyeonggi-do 11426 (KR); SIM, Sehoon, Suwon-si, Gyeonggi-do 16493 (KR); KIM, Hongman, Suwon-si, Gyeonggi-do 16438 (KR); SONG, Wooseok, Seoul 05698 (KR); JUN, Hyeon, Seoul 08763 (KR)
(74) Representative: Dehns
(86) International application number: PCT/KR2023/007006
(87) International publication number: WO 2023/229344

(57) **Abstract**

The present invention relates to a gene carrier including a nanoparticle, oligo Ts bound to the surface of the nanoparticle, and for delivering polyadenylated nucleic acid molecules derived from a target gene, wherein the gene carrier has the effect of efficiently delivering the target gene into a cell without going through an additional processing step for binding the target gene and the carrier.

## Description

### [Technical Field]

The present invention relates to a gene carrier comprising nanoparticles, oligo Ts bound to the surface of the nanoparticles, and polyadenylated nucleic acid molecules derived from a target gene for delivery.

### [Background Art]

A gene is an organic material present in all living organisms, containing all the information necessary for the composition, maintenance, and organic interactions of cells in the organism. Genes can be damaged by various factors, and such damage may develop into a wide range of diseases.

Gene therapy, which involves introducing foreign genes (DNA or RNA) required by the patient to treat diseases caused by gene damage, is under investigation. All living organisms possess defense mechanisms against foreign genes. Therefore, the development of an effective gene delivery system is essential for efficient gene therapy. To date, gene therapeutics have primarily relied on viral vector technology. However, this approach has critical drawbacks, such as eliciting strong immune responses and limitations in the range of applicable genes. To overcome the limitations of viral vector technology, non-viral vector technologies, including cationic lipids, polymers, dendrimers, and peptides, have been developed. While non-viral vector technologies offer improved safety compared to viral vector systems, they exhibit reduced gene delivery efficiency.

Therefore, there is a need for a new gene delivery technology that can satisfy both safety and efficiency. Recently, non-viral vector technologies utilizing nanoparticles, including carbon nanotubes, iron oxide, silica, and gold, have been studied as alternative solutions.

To date, more than half of gene therapy studies have utilized viral vector technology. Viral vector technology employs the replication mechanisms inherent to viruses as gene carriers, with representative examples including adenoviruses, retroviruses, and adeno-associated viruses (AAV). While viral vector technology offers high gene delivery efficiency, it poses significant safety concerns due to the pathogenic nature of viruses, such as inducing strong immune responses. Additionally, there are limitations on the size of genes that can be inserted into viral vectors, restricting the range of applicable genes.

In contrast, non-viral vector technologies, such as plasmids and liposomes, offer several advantages. Non-viral vectors do not introduce viral genetic material into human cells, are easier to produce, allow for the insertion of genes of unlimited size, and elicit minimal immune responses in the host, resulting in fewer side effects. However, they have the disadvantage of lower intracellular delivery efficiency compared to viral vectors.

To address the limitations of existing gene carriers, recent research has focused on nanoparticles, including carbon nanotubes, iron oxide, silica, and gold, as alternatives for gene delivery technologies. Among these, gold nanoparticles are particularly attractive scaffolds for developing gene delivery systems due to their bioinertness, non-toxicity, ease of synthesis, and functionalization. To date, several gene delivery systems have been developed, including gold nanoparticles protected by mixed monolayers, polymer-gold nanoparticle complexes, gold nanoparticles functionalized with double-stranded DNA, and gold nanoparticles functionalized with single-stranded DNA.

These nanoparticles have demonstrated lower cytotoxicity to cells, higher gene delivery efficiency, and improved resistance to nucleases compared to commonly used gene delivery systems such as Lipofectamine and Cytofectin. However, these systems are limited to delivering genes covalently bound to the nanoparticles. This necessitates the individual synthesis of nanoparticles for each target gene, requiring additional processes for their synthesis, which presents a significant inconvenience.

To overcome these drawbacks, the inventors developed a novel gene delivery system by introducing a universal binding partner covalently attached to the surface of nanomaterials, which can bind to a modified gene carrying a complementary binding counterpart (Patent No. 10-2011-0050338). However, this system requires an additional process to modify the gene to have a complementary sequence to the universal binding partner. Such modifications, which involve adding nucleotide sequences to the gene, may reduce translation efficiency or alter the translation product. Therefore, there is a need for research into gene carriers that can be utilized more conveniently without requiring additional modifications to the gene.

### [Disclosure]

### [Technical Problem]

In response, the inventors of the present invention have completed the present invention for a carrier including polyadenylated genes, which enables binding of a gene to the gene to be delivered without an additional processing step so that the gene has a base sequence complementary to the universal binding partner, through a gene delivery system in which Oligo T sequences, a universal binding partner, are introduced by covalent bonding to the surface of a nanomaterial.

Therefore, the present invention is directed to providing a gene carrier which includes a nanoparticle, Oligo T conjugated to the surface of the nanoparticle, and polyadenylated nucleic acid molecules.

### [Technical Solution]

To address the above-described objects, the present invention provides a gene carrier, which includes a nanoparticle, Oligo T conjugated to the surface of the nanoparticle, and polyadenylated nucleic acid molecules.

Hereinafter, the present invention will be described in detail.

The present invention relates to a gene carrier, for delivering a gene such as mRNA to a target, the gene carrier comprising a nanoparticle and Oligo T sequences covalently linked to the surface of the nanoparticle, and polyadenylated nucleic acid molecules binding to the Oligo T.

Diseases caused by gene deficiency or damage can be treated with gene therapy technology that replaces or corrects the deficient/damaged gene by introducing a foreign gene. However, since all organisms have a defense mechanism against the introduction of foreign genes, an effective gene delivery system needs to be developed to deliver foreign genes into organisms. The gene carrier of the present invention is non-toxic and can efficiently deliver foreign genes into cells, and has been developed to control the expression of target genes without affecting normal cells.

That is, the gene carrier of the present invention has been developed to introduce polyadenylated nucleic acid molecules, such as oligo T that can bind to mRNA, by covalent bonding to the surface of nanoparticles, and to deliver the nucleic acid molecule to be expressed to the target cell by binding to oligo T. That is, the present invention is a technology that can deliver the gene to be introduced without additional modification or processing by using oligo T that targets the polyadenylated tail (poly A tail) naturally present in nucleic acid molecules.

In the present invention, the 'nanoparticle (NP)' refers to a material having a size preferably of 1-100 nm. The nanoparticles used in the present invention are not particularly limited in form and shape as long as they are substances having a nano-size (for example, in the form of particles, tubes, rods, or regular tetrahedrons). According to a preferred embodiment of the present invention, the "nanoparticles" refer to particles of various substances having a diameter in the nano-unit, preferably 8-100 nm, more preferably 10-50 nm, and most preferably 12-14 nm. The nanoparticles are not particularly limited as long as they are particles having a nano-size.

According to the most preferred embodiment of the present invention, the nanoparticles used in the present invention refer to gold nanoparticles. Gold nanoparticles are not only easy to manufacture in the form of stable particles, but can also be varied in size from 0.8 nm to 200 nm to suit various purposes. In addition, gold can modify its structure by combining with various types of molecules, such as peptides, proteins, and nucleic acids, and reflects light at various wavelengths, which can be used to easily identify its location within a cell. In addition, gold nanoparticles are harmless to the human body and have high biocompatibility, unlike heavy metals such as manganese, aluminum, cadmium, lead, mercury, cobalt, nickel, and beryllium.

When the diameter of the above gold nanoparticles becomes larger than 100 nm, not only does the characteristic of the nanoparticle disappear, but also the bond between the gold surface without the characteristic of the nanoparticle and the functional group such as the thiol group becomes weak, making it difficult to manufacture particles in which oligo T is combined via the gold nanoparticle. Therefore, the diameter of the gold nanoparticles of the present invention is not limited thereto, but may be preferably 8-100 nm, more preferably 10-50 nm, and most preferably 12-14 nm.

In one embodiment, the gold nanoparticle used in the present invention may be prepared as follows: HAuCl₄ is used as a gold source, sodium citrate is used as a reducing agent to reduce HAuCl₄, thereby preparing a gold nanoparticle. In this case, the size of the gold nanoparticle can be controlled by changing the amount of citrate added. That is, as the amount of citrated added increases, the size of the metal nanoparticle decreases due to more nucleation.

In the present invention, 'Oligo T' for binding to polyadenylated nucleic acid molecules has additional functionality in order to be covalently bonded to the surface of the nanoparticles. The additional functionality is located at the end of the oligonucleotide. More preferably, the oligo T has a thiol group or an amine group bonded to the terminal, and even more preferably, the additional functionality is located at the 5'-terminus.

As described above, a nucleic acid molecule may be directly bound to a portion that imparts functionality, but preferably, a 'linker (or spacer)' may intervene in the middle. The linker enables the oligo T to be bound to the nanoparticle at a high density and serves to facilitate binding to a target gene (e.g., mRNA). The linker connects the oligo T and the target gene (e.g., mRNA), and is not limited to a type thereof, but may be an oligonucleotide or a polyether, and preferably may include at least one linker of an oligo A nucleotide, an oligo T nucleotide, an oligo G nucleotide, or an oligo C nucleotide.

According to a preferred embodiment of the present invention, the oligo T may have a structure of "5'-SH (or NH₂)-linker-oligo T nucleotide-3' ".

In the present invention, the oligo Ts are not particularly limited in length, but may preferably contain 3 to 100 Ts, more preferably 10 to 50 Ts, and even more preferably 15 to 30 Ts (Thymine).

The oligo T can bind to the polyadenylation (poly A) sequence of a target gene to be delivered to a cell, for example, mRNA, and the polyadenylation sequence is not limited in number, but may include from about 20 to several hundred A (Adenines), and preferably may include about 20 to 250 A. The polyadenylation is added in the post-transcription process of mRNA, and the gene carrier of the present invention can particularly include polyadenylated mRNA as a nucleic acid molecule for delivery, and has the characteristic of being able to deliver mRNA to a target cell.

As an example, the nucleic acid molecule derived from the target gene can be an expression construct comprising a promoter for expressing the same, a coding sequence of the target gene operatively linked to the promoter, and a polyadenylation sequence. The term "operably linked" as used herein means a functional linkage between a nucleic acid expression control sequence regulatory sequence (e.g., a promoter, a signal sequence, or an array of transcription factor binding sites) and another nucleic acid sequence, whereby the control sequence can control the expression of the other nucleic acid sequence.

The target gene may be a gene to be expressed in a cell, particularly an RNA or DNA, and more preferably an mRNA molecule comprising a polyadenylation sequence. This may include a human-derived or exogenous gene (e.g., a virus), or a therapeutic transgene such as a tumor suppressor gene, an antigenic gene, a cytotoxic gene, a cell proliferation inhibitory gene, an apoptotic gene, and an angiogenesis inhibitory gene. In one embodiment of the present invention, the target gene is an antigenic gene, and the intracellular delivery ability was confirmed using mRNA derived from the RBD of SARS-CoV2.

In the present invention, the gene carrier can be used as a gene carrier capable of efficiently delivering a foreign gene into a cell and inducing expression, and can be utilized as a drug delivery agent in a medicine or vaccine using the mRNA molecule itself.

That is, as another aspect of the present invention, the present invention provides a use of the gene carrier that can be applied as a component of a gene therapy agent, a cell therapy agent, or a vaccine. The gene carrier of the present invention can be used as a pharmaceutical composition for the purpose of gene delivery.

### [Advantageous Effects]

The present invention relates to a gene carrier that includes a nanoparticle, OligoTs linked to the surface of the nanoparticle, and for delivering polyadenylated nucleic acid molecules derived from a target gene. The gene carrier of the present invention can be used universally without going through a separate processing process for binding the target gene and the carrier, and has the effect of efficiently delivering the target gene into a cell. In addition, compared to the prior art AuNP^{RNAI} nucleic acid delivery vehicle, not only is the binding ability to the nucleic acid to be delivered increased, but the intracellular delivery efficiency is also increased.

### [Description of Drawings]

FIG. 1 is a schematic diagram of a gold nanoparticle-based gene delivery system functionalized with oligoT that specifically binds to the polyadenylation sequence of mRNA.
FIG. 2 is a transmission microscope analysis image of the synthesized gold nanoparticles.
FIG. 3 shows an 8M Urea 10% polyacrylamid gel electrophoresis analysis image of AuNP^{dT} manufactured in the present invention.
FIG. 4 is a fluorescence microscope image of HeLa cells delivered with AuNP^{dT}-oligoA manufactured in the present invention.
FIG. 5 is an image of 8M Urea 10% polyacrylamid gel electrophoresis analysis of AuNP^{dT}-GFP mRNA, AuNP^{RNAI}-GFP mRNA (A), AuNP^{dT}-SARS-CoV-2 RBD mRNA, and AuNP^{RNAI}-SARS-CoV-2 RBD mRNA (B) manufactured in the present invention.
FIG. 6 is a drawing showing the results of confirming GFP mRNA delivery by AuNP^{dT} in a mouse xenograft model using immunohistochemical staining.
FIG. 7 is a drawing showing the results of confirming SARS-CoV-2 RBD mRNA delivery by AuNP^{dT} in rats using ELISA analysis.

### [Best modes of the Invention]

Hereinafter, the present invention will be described in detail with reference to examples to help in understanding the present invention. However, examples according to the present invention may be modified in a variety of different forms, and it should not be construed that the scope of the present invention is limited to the following examples. The examples of the present invention are provided to more completely explain the present invention to those of ordinary skill in the art.

### Example 1. Preparation of gold nanoparticles (AuNP^{dT}) functionalized with oligo T

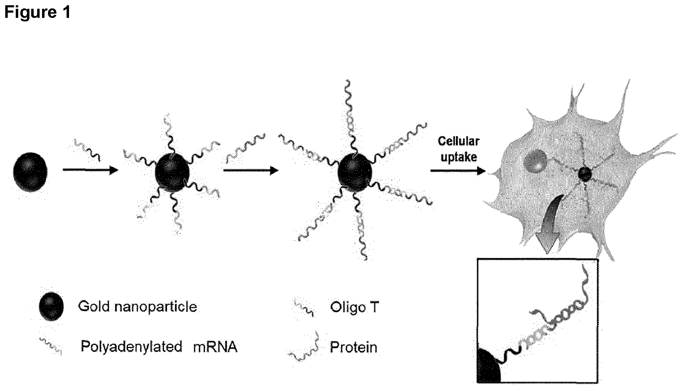

A gene carrier was prepared by covalently bonding oligo T (Oligo T) to gold nanoparticles to bind to the polyadenylation sequence (poly A) of messenger RNA, and the schematic diagram of the manufacturing process is as shown in FIG 1.

### 1-1. Preparation of 13nm Gold Nanoparticle.

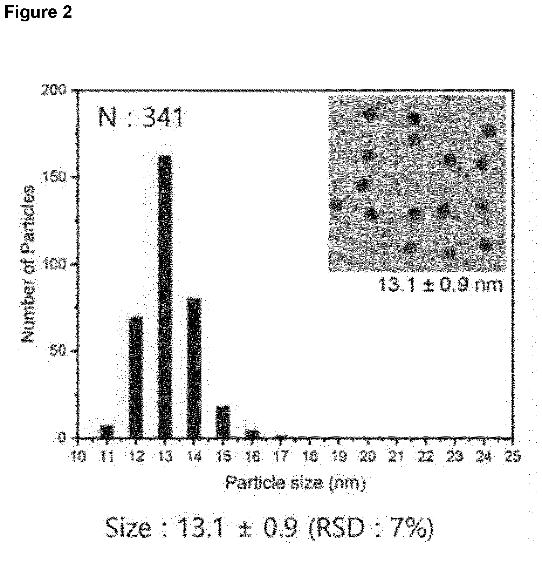

The gold nanoparticles used in the present invention used HAuCl₄ as a gold source, and the HAuCl₄ was reduced using sodium citrate as a reducing agent to prepare the gold nanoparticles. More specifically, 545 ml of a 0.92 mM HAuCl4 solution and 5 ml of a 388 mM sodium citrate solution were mixed, and then reacted at 100°C for 15 minutes. The reaction product was analyzed using a transmission electron microscope to confirm whether nanoparticles were synthesized and their size (FIG. 2).

### 1-2. Pretreatment of oligoT

In order to produce a gene carrier according to the present invention, an OligoT sequence that detects and specifically binds to a polyadenylation sequence of mRNA was used. More specifically, the OligoT is an oligonucleotide whose 5'-terminal is modified with a thiol group, and the OligoT of SEQ ID NO: 1 ((SH) 5' - TTTTTTTTTTTTTTTTTTTTTTTTTT - 3') was used. The dried OligoT was dissolved in water to a final concentration of 100 µM, and 20 µl of 3 M sodium acetate (pH 5.2) and 30 µl of 1 N DTT (dithiothreitol) were added to 150 µl of the oligo, and the mixture was reacted at room temperature for 60 minutes. In order to remove the DTT including the unbound thiol group, 200 µl of ethyl acetate was mixed, and the supernatant was removed by centrifugation, and this process was repeated three times. Then, the oligo T was precipitated using the EtOH precipitating method.

### 1-3. Preparation of gold nanoparticles (AuNP^{dT}) functionalized with oligoT

The oligoT precipitated by pretreatment as in Example 1-2 was dissolved in water, added to the gold nanoparticles synthesized in Example 1-1, and then combined using the salt aging method.

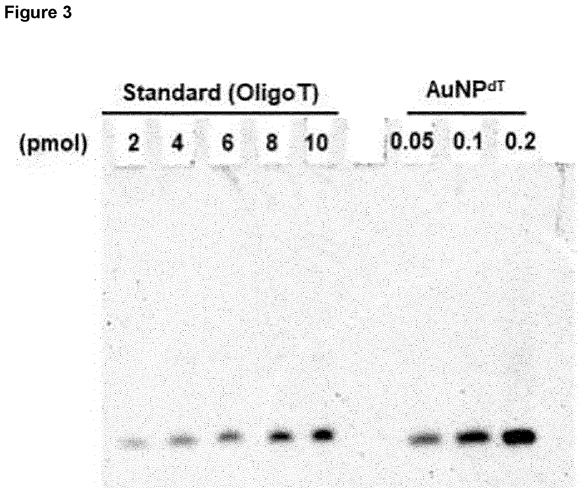

Specifically, oligo T was added to 5-50 nM gold nanoparticles (AuNP:OligoT ratio = 1:100-300) and mixed sufficiently, then NaCl was added to a concentration of 0.1-1.0 M and mixed for 4 hours. After 4 hours, NaCl was added to double the concentration and mixed for 4 hours. After 4 hours, NaCl was added to a concentration of 0.3 M and mixed for 12 hours. After 12 hours, the oligo T and gold mixture was centrifuged at ~10,000*g for 20 minutes, collected, and unreacted oligo T in the supernatant was removed. This process was repeated three times. The final gold nanoparticle-Oligo T complex was dispersed in 10 mM sodium phosphate buffer (pH 7.4) containing 0.1 M NaCl. The manufactured gold nanoparticle-oligo T complex was analyzed by electrophoresis using 10% acrylamide 8 M urea gel, and it was confirmed that 130 to 150 oligo T complexes were bound to one gold nanoparticle (FIG. 3).

### Example 2. Confirmation of gene transfer ability by AuNP^{dT} in vitro

### 2-1. Preparation of AuNP^{dT}-oligo A conjugate

Oligo A (5'-AAAAAAAAAAAAAAAAAAAA, sequence number 2), the polyadenylation sequence of mRNA, was labeled with Cy3 to confirm the intracellular transfer ability of the polyadenylated nucleic acid molecule by AuNP^{dT} in-vitro.

To prevent the formation of secondary structures of nucleic acid molecules, Oligo A was first pre-incubated at 80°C for 5 minutes. After mixing the Oligo A and AuNP^{dT} in 10 mM sodium phosphate buffer (pH 7.4) containing 0.1 M NaCl, the mixture was reacted at 55°C for 10 minutes and then at 4°C for about 30 minutes. The resulting conjugate was centrifuged at 15,000*g for 10 minutes, the supernatant was removed, and binding was confirmed by electrophoresis on a 10% acrylamide 8 M urea gel.

### 2-2. Confirmation of intracellular delivery ability of AuNP^{dT}-oligoA

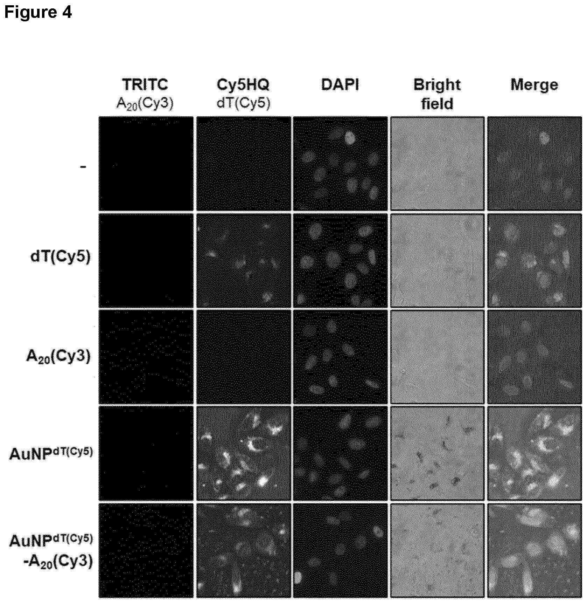

In order to confirm the intracellular delivery ability of AuNP^{dT}-oligoA produced in 2-1 above, an experiment was conducted targeting HeLa (Human cervical carcinoma) cells. HeLa cells were cultured in Dulbecco's modified Eagle's medium (DMEM) containing 10% heat-inactivated fetal bovine serum (Caisson, USA) and 1% penicillin-streptomycin (Welgene, Korea) in a 5% CO₂, humidity-controlled 37°C incubator. Approximately 4 × 10⁴ cells were plated in a 24-well plate in the medium containing 10% FBS to obtain 50 to 60% confluence. The AuNP^{dT}-oligoA prepared in 2-1 above was added to the HeLa cell culture medium to a final concentration of 1 nM, and the fluorescence emitted from the cells was measured using a fluorescence microscope after 24 hours. As a result, as shown in FIG. 4, it was confirmed that the intracellular transport of nucleic acid molecules including oligo A was further increased when the fabricated gold nano carrier was used.

### Example 3. Confirmation of gene transfer ability by AuNP^{dT} in small animal model

An animal experiment was conducted to confirm the target gene transfer ability of the gold nanoparticle gene carrier of the present invention. AuNP^{dT}-GFP mRNA and AuNP^{dT}-SARS-CoV-2 RBD mRNA were injected into mice and rats, respectively, to confirm the transfer ability. The animals were maintained in an animal breeding room maintained at 30 to 40% humidity and 22±1°C temperature. The lighting in the room was a 12-h light/dark cycle. The animal protocol was approved by the Support Center for Animal Experiments at Chung-Ang University, and the animals were treated as described in the protocol.

### 3-1. In-vitro synthesis of mRNA

Messenger RNA was synthesized from DNA templates of SEQ ID NO: 3 and SEQ ID NO: 4 containing the T7 promoter sequence and subsequent gene sequences using MEGA shortscriptTM kit (Ambion, USA) and CleanCap AG (Trilink, USA) and N1-Methylpseudo-UTP (JenaBioscience, Germany) according to the manufacturer's instructions.

The DNA template for GFP mRNA synthesis was composed of the base sequence of SEQ ID NO: 3 (including the T7 promoter), and the DNA template for SARS-CoV-2 RBD mRNA synthesis was composed of the base sequence of SEQ ID NO: 4. The synthesized mRNA was purified using the phenol extraction method.

### 3-2. Preparation of AuNP^{dT}-mRNA conjugate

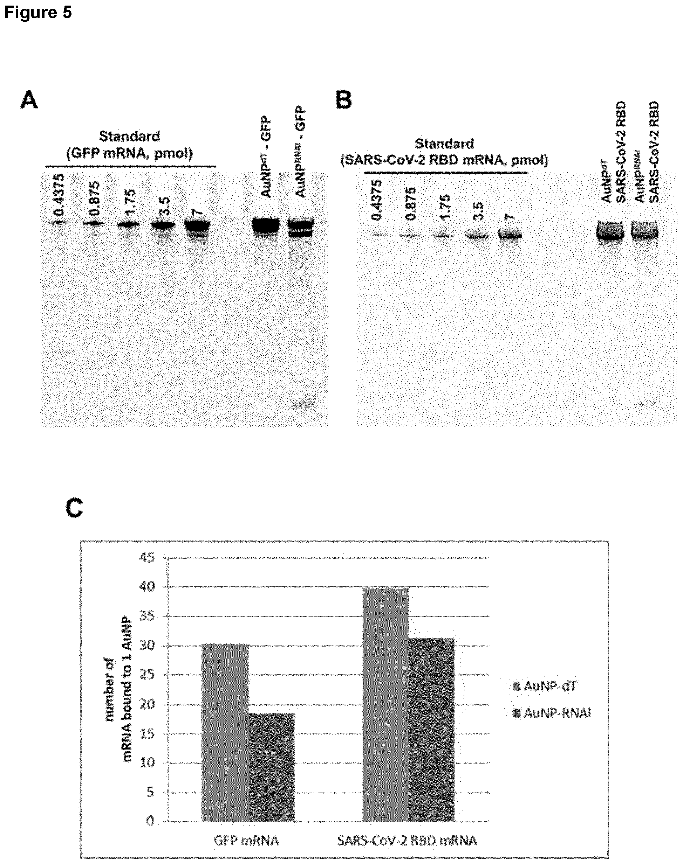

AuNP^{dT} and GFP mRNA and SARS-CoV-2 RBD mRNA conjugates were prepared in the same manner as in Example 2-1 above. The manufactured AuNP^{dT}-mRNA conjugates were analyzed by electrophoresis on a 10% acrylamide 8M urea gel, and it was confirmed that 30 to 40 mRNAs were bound to one gold nanoparticle in each conjugate (FIG. 5). Compared to AuNP^{RNAI}, a technology of a prior patent application (Korean Patent No. 10-2011-0050338), it was confirmed that the mRNA binding ability of the AuNP^{dT} of the present invention was increased by more than 30%.

### 3-3. Confirmation of the biological delivery ability of AuNP^{dT}-GFP mRNA in mice

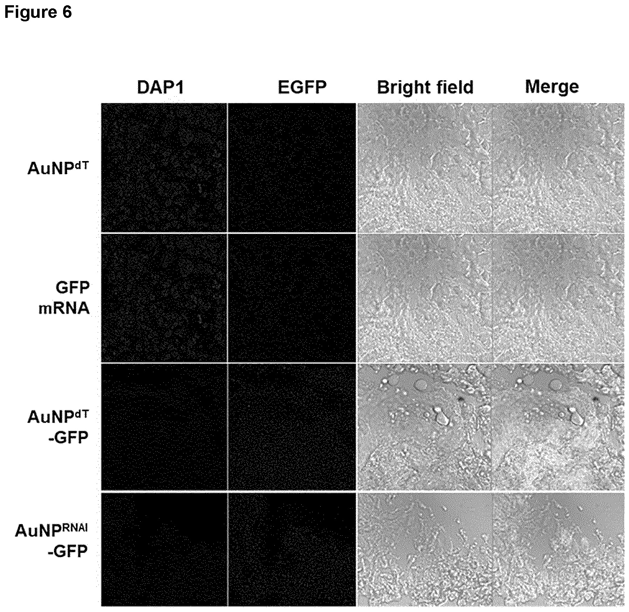

For the convenience of the experiment, a xenograft tumor model was used. HeLa cells were injected into 6-week-old immunodeficient BALB/c-nu/nu mice (Central Lab Animal Inc, Korea) to induce cervical cancer, and AuNP^{dT}-GFP mRNA was injected into the xenograft tumors three times at 24-hour intervals. The xenograft tumors were extracted and the expression of GFP mRNA was measured by immunohistochemical staining (FIG. 6). As a result, GFP protein expression was confirmed in the tumors injected with AuNP^{dT}-GFP mRNA, confirming the intracellular movement of the AuNP^{dT}-GFP mRNA. In addition, it was confirmed that the AuNP^{dT} of the present invention has a superior mRNA delivery ability when compared to AuNP^{RNAI}, a technology of a prior patent application (Korean Patent No. 10-2011-0050338).

### 3-4. Confirmation of the biotransferability of AuNP^{dT}-SARS-CoV-2 RBD mRNA in rats

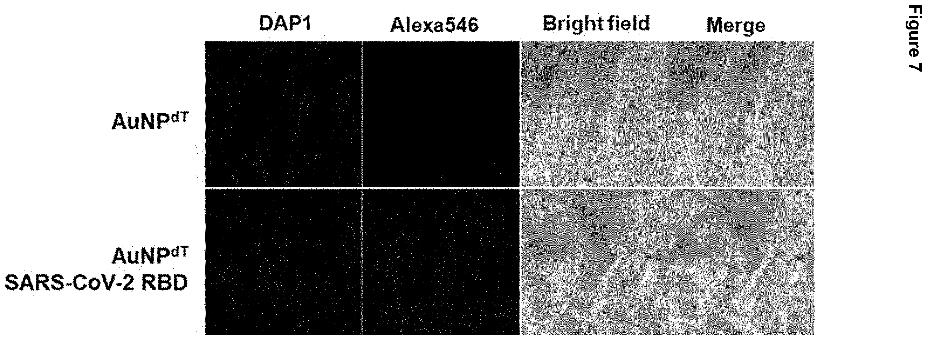

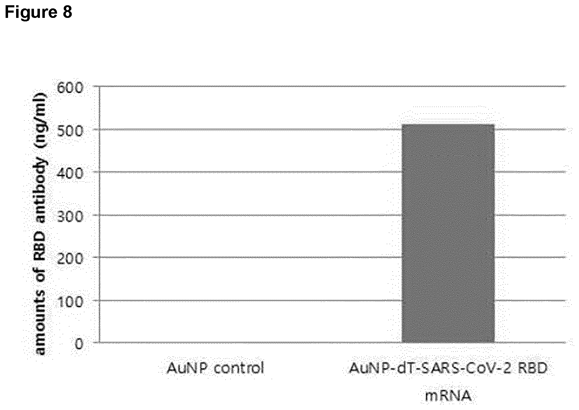

AuNP^{dT}-SARS-CoV-2 RBD mRNA was injected intramuscularly three times at two-week intervals into 6-week-old Sprague-Dawley rats (Samtako, Korea). The muscles at the injection site were extracted and the expression of SARS-CoV-2 RBD mRNA was measured by immunohistochemical staining (FIG. 7). The serum of the rats was extracted and the formation of antibodies against the RBD protein was measured by ELISA (FIG. 8). As a result, as shown in FIG. 7, it was confirmed that RBD protein was expressed in the muscle tissue injected with AuNP^{dT}-SARS-CoV-2 RBD mRNA, indicating that the AuNP^{dT}-SARS-CoV-2 RBD mRNA was translocated into cells in vivo. In addition, in this regard, as shown in FIG. 8, by confirming that RBD antibodies were formed in the serum of rats injected with AuNP^{dT}-SARS-CoV-2 RBD mRNA, it was possible to confirm the expression of mRNA that had moved into the cells.

In the above, the present invention was described with reference to embodiments. It will be understood by those of ordinary skill in the art that the present invention can be implemented in modified forms without departing from the essential features of the present invention. Therefore, the disclosed embodiments should be considered from a descriptive perspective, rather than a restrictive perspective. The scope of the present invention is shown in the claims rather than the foregoing description, and all differences within the equivalent range thereto will be construed as being included in the present invention.

### [Modes of the Invention]

In one aspect of the present invention, the present invention relates to a gene carrier comprising a nanoparticle; Oligo Ts covalently bonded to the surface of the nanoparticle; and nucleic acid molecules comprising polyadenylation sequences bound to the Oligo Ts.

In the present invention, the nanoparticle is characterized as a nanoparticle having a size of 8-100 nm.

In the present invention, the nanoparticle is a gold nanoparticle (AuNP).

In the present invention, the Oligo Ts have a thiol group or a amine group bonded to the terminal.

In the present invention, the Oligo Ts are composed of 3 to 100 thymines.

In the present invention, the nucleic acid molecule comprising the polyadenylation sequences may be RNA or DNA.

In the present invention, the nucleic acid molecule comprising the polyadenylation sequence is mRNA.

In the present invention, the nucleic acid molecule may be derived from one or more genes selected from the group consisting of a tumor suppressor gene, an antigenic gene, a cytotoxic gene, a cell proliferation inhibitory gene, a cell death gene, and an angiogenesis inhibitory gene.

In the present invention, the nucleic acid molecule may be an antigenic gene derived from a virus.

## Claims

1. A gene carrier, comprising:
a nanoparticle;
Oligo Ts covalently bonded to the surface of the nanoparticle; and
nucleic acid molecules comprising polyadenylation sequences bound to the Oligo Ts.

2. The gene carrier of claim 1, wherein the nanoparticle is characterized as a nanoparticle having a size of 8-100 nm.

3. The gene carrier of claim 1, wherein the nanoparticle is a gold nanoparticle (AuNP).

4. The gene carrier of claim 1, wherein the Oligo Ts have a thiol group or a amine group bonded to the terminal.

5. The gene carrier of claim 1, wherein the Oligo Ts are composed of 3 to 100 thymines.

6. The gene carrier of claim 1, wherein nucleic acid molecules comprising the polyadenylation sequences may be RNA or DNA

7. The gene carrier of claim 1, wherein the nucleic acid molecules comprising the polyadenylation sequence is mRNA.

8. The gene carrier of claim 1, wherein the nucleic acid molecules may be derived from one or more genes selected from the group consisting of a tumor suppressor gene, an antigenic gene, a cytotoxic gene, a cell proliferation inhibitory gene, a cell death gene, and an angiogenesis inhibitory gene.

9. The gene carrier of claim 8, wherein the nucleic acid molecule may be an antigenic gene derived from a virus.
